# EUROPÄISCHE PATENTANMELDUNG

(11) **EP 4 809 950 A2**
(43) Veröffentlichungstag der Anmeldung: **23.09.2026**
(21) Anmeldenummer: 26195565.2
(22) Anmeldetag: 23.07.2019
(51) Int. Cl.: A61B 18/02

(54) **KRYOSONDE**

(62) Dokumentnummer(n) der früheren Anmeldung(en) nach Art. 76 EPÜ: 19187779.4 / 3 769 706
(71) Anmelder: Erbe Elektromedizin GmbH, 72072 Tübingen (DE)
(72) Erfinder: Adler, Marcus, 72393 Burladingen (DE); Kronenthaler, Jorg, 72145 Hirrlingen (DE)
(74) Vertreter: Rüger Abel IP Legal Solutions GmbH

(57) **Zusammenfassung**

Eine erfindungsgemäße Kryosonde weist einen Kopf (20) auf, dem über ein Kapillarrohr (22a) Kryofluid zugeführt wird. Zur Abfuhr des Kryofluids dient ein Schlauch (18), durch dessen Lumen (21) sich das Kapillarrohr (22a) erstreckt. Das Kapillarrohr (22a) weist einen biegeweichen Abschnitt (55) auf, der von einem Zugelement (26) überbrückt wird. Auf diese Weise wird eine gut handhabbare Kryosonde (11) erhalten, die sich leicht und sehr weit abwinkeln lässt und dennoch die zur Probeentnahme nötigen Zugkräfte überträgt.

## Beschreibung

Die Erfindung betrifft eine Kryosonde, die insbesondere zur Gewebeprobenentnahme in stark verzweigten Gefäßsystemen, wie beispielsweise zur Gewebeprobeentnahme im oberen Harntrakt, speziell im Nierenkelchsystem, geeignet ist.

Kryosonden für die Gewebebiopsie sind prinzipiell bekannt. Zum Beispiel offenbart die DE 10 2011 000 004 B4 eine flexible Kryosonde mit einem Schlauch, der ein Lumen aufweist und an dessen distalen Ende ein metallischer Kopf in Gestalt eines Bechers angeordnet, dessen Durchmesser mit dem Durchmesser des Schlauchs übereinstimmt und der an seinem distalen Ende einen gerundeten Boden aufweist. In dem Schlauch ist ein Kapillarrohr angeordnet, das sich durch die gesamte Länge des Schlauchs erstreckt und innerhalb des Kopfs endet. Das Kapillarrohr dient zum Einleiten von Kryofluid in den Kopf, um diesen zu kühlen.

Ähnliche Kryosonden sind aus der EP 2 257 235 B1, der EP 2 170 197 sowie der EP 2 114 276 B1 bekannt. Zum Beispiel offenbart die EP 3 323 366 A1 eine Kryosonde mit einem zweilumigen Schlauch, auf dessen distalen eine Metallkappe gehalten ist. In einem der Lumen steckt eine Düse, über die Kryofluid in einen von der Kappe umschlossenen Innenraum geleitet wird. Das andere Lumen dient der Rückleitung von Fluid. Außerdem offenbart die US 6 270 475 B1 ein Instrument mit einem hohlen Schaft, durch dessen Lumen sich elektrische Leitungen erstrecken.

Bei der Gewebeprobenentnahme wird das distale Ende der Sonde soweit abgekühlt, dass das zu entnehmende Gewebe an dem Kopf der Sonde anfriert. Das Gewebe muss dann von dem nicht gefrorenen Gewebe getrennt, d.h., abgerissen und mit der Sonde aus dem Lumen des Patienten herausgeführt werden. Bei verwinkelten Lumen kommt es dabei zusätzlich darauf an, die Sonde mit geringem Biegeradius abwinkeln zu können, wobei eine Abwinkelung von deutlich mehr als 90° gewünscht sein kann.

Davon ausgehend ist es Aufgabe der Erfindung, eine Kryosonde bereitzustellen, die für derart anspruchsvollen Gebrauch geeignet ist.

Diese Aufgabe wird mit der Kryosonde nach Anspruch 1 erfüllt.

Die erfindungsgemäße Kryosonde umfasst einen Schlauch, der an seinem Ende mit einem Kopf versehen ist, der zur Gewebeentnahme dient. Durch das Lumen des Schlauchs erstreckt sich ein Kapillarrohr, um den Kopf mit einem Kryofluid zu versorgen. Das Kapillarrohr ist eine Leitung, deren Druckfestigkeit auf das einzusetzende Kryofluid abgestimmt ist.

In dem Lumen des Schlauchs ist zusätzlich ein Zugdraht angeordnet, der der Übertragung von Zugkräften von dem Kopf auf ein proximales Ende der Kryosonde dient. Dies ermöglicht die Nutzung eines in einem oder mehreren Abschnitten oder insgesamt hochflexiblen Kapillarrohrs und andererseits auch die Übertragung der nötigen Zugkräfte von dem proximalen Ende auf den Kopf, um an dem Kopf angefrorenes Gewebe von nicht gefrorenem Gewebe abzureißen und so eine Gewebeprobe zu entnehmen.

Die erfindungsgemäße Kryosonde ist so flexibel, dass sie mit geringem Krafteinsatz in großen Winkeln abgewinkelt werden kann, und sie vereint dies mit hoher Zugfestigkeit.

Die Kryosonde kann sehr filigran gestaltet werden und einen Außendurchmesser von weniger als 1,2 mm aufweisen, sodass sie auch in sehr engen Endoskopen einsetzbar ist. Es können mit dem erfindungsgemäßen Konzept Biegewinkel, d.h., ein Abwinkeln von mehr als 150° vorzugsweise mehr als 160° erreicht werden. Dabei kann aufgrund der hohen Flexibilität der Kryosonde das Abwinkeln mit geringem von dem Endoskop aufbringbaren Kraftaufwand durchgeführt werden.

Vorzugsweise ist das Kapillarrohr aus einem Material gefertigt, bei dem durch eine Einwirkung, beispielsweise eine Wärmebehandlung, eine Erhöhung oder eine Verminderung seiner Zugfestigkeit erzielbar ist. Weiter vorzugsweise ist das Kapillarrohr so behandelt oder bearbeitet, dass es wenigstens einen Abschnitt aufweist, in dem die Zugfestigkeit des Materials und somit auch die Biegesteifigkeit des Kapillarrohrs gegenüber der Zugfestigkeit und somit auch der Biegesteifigkeit des übrigen Kapillarrohrs reduziert ist. Dieser Abschnitt reduzierter Zugfestigkeit und reduzierter Biegesteifigkeit ist vorzugsweise in demjenigen distalen Abschnitt der Kryosonde angeordnet, der von dem Endoskop im Einsatz abgewinkelt wird. Das Kapillarrohr besteht beispielsweise aus Stahl oder einem anderen zugfesten und biegesteifen Material. In dem Abschnitt, in dem die Sonde mit geringem Biegeradius abzuwinkeln ist, kann der Stahl beispielsweise weichgeglüht sein. Alternativ ist es möglich, diesen Abschnitt durch ein Kapillarrohrstück aus anderem Material zu bilden, beispielsweise Kunststoff, Kupfer oder dergleichen.

Der Zugdraht dient zur Übertragung von Zugkräften von dem proximalen Ende auf den Kopf der Sonde. Er ist wenigstens den weichen Abschnitt des Kapillarrohrs überspannend angeordnet. Dazu ist der Zugdraht an dem distalen Ende zugfest mit dem Kopf des Instruments verbunden. Der Zugdraht kann dazu direkt an den Kopf oder auch an einen zugfesten Abschnitt des Kapillarrohrs angeschlossen sein, das wiederum mit dem Kopf verbunden sein kann.

Das distale Ende des Zugdrahts kann bis zum distalen Ende der Sonde oder, wie es bevorzugt wird, mit dem zugfesten distalen Teil des Kapillarrohrs verbunden sein. Damit überspannt der Zugdraht wenigstens den Abschnitt der Kapillare der eine reduzierte Biegesteifigkeit aufweist.

Der Zugdraht kann beispielsweise über Schweißpunkte, Schweißnähte oder andere Verbindungsarten, zugfest mit dem Kapillarrohr verbunden sein. Vorzugsweise liegt der Zugdraht dabei parallel zu dem Kapillarrohr, d.h., die Verbindungsstellen zwischen Zugdraht und Kapillarrohr sind bezüglich des Kapillarquerschnitts in gleicher Winkelposition angeordnet. Dadurch wird eine freie Winkelbewegung der Sonde in allen Radialrichtungen ermöglicht.

Obwohl es prinzipiell möglich ist, den biegeweichen Abschnitt des Kapillarohrs mit mehreren Zugdrähten zu überbrücken, wird es bevorzugt, lediglich einen Zugdraht vorzusehen. Es ergibt sich dabei eine gute Beweglichkeit der Kryosonde in allen Richtungen.

Der Durchmesser des Zugdrahts ist vorzugsweise geringer als die Differenz zwischen dem Durchmesser des Lumens und dem Außendurchmesser des Kapillarohrs. Damit bleibt sowohl das Kapillarrohr als auch der Zugdraht in dem Lumen radial und in Umfangsrichtung beweglich. Bei einer Biegung der Sonde, bei der der Zugdraht bezüglich des Biegeradius radial außen liegt, kann zumindest der mittlere Abschnitt des Zugdrahts in dem Lumen wandern und auf die gegenüberliegenden Seite des Lumens gelangen. Dadurch setzt der Zugdraht auch in diesem Fall einer Abwinklung keinen Widerstand entgegen.

Weitere Einzelheiten vorteilhafter Ausführungsformen der Erfindung ergeben sich aus der Zeichnung, der Beschreibung und Ansprüchen. Es zeigen:
Figur 1 ein Endoskop mit einer erfindungsgemäßen Sonde, in schematisierter perspektivischer Darstellung,
Figur 2 das distale Ende des Endoskops mit Sonde, in abgewinkeltem Zustand,
Figur 3 das distale Ende der Sonde, in schematischer längsgeschnittener Darstellung,
Figur 4 einen Ausschnitt des distalen Endes der Sonde, in schematisierter längsgeschnittener Darstellung,
Figur 5 und 6 Schnittdarstellungen der Sonde nach Figur 4, geschnitten entlang der Linien V-V bzw. VI-VI,
Figur 7 eine alternative Ausführungsform des Instruments,
Figur 8, das Instrument nach Figur 7 im Querschnitt.

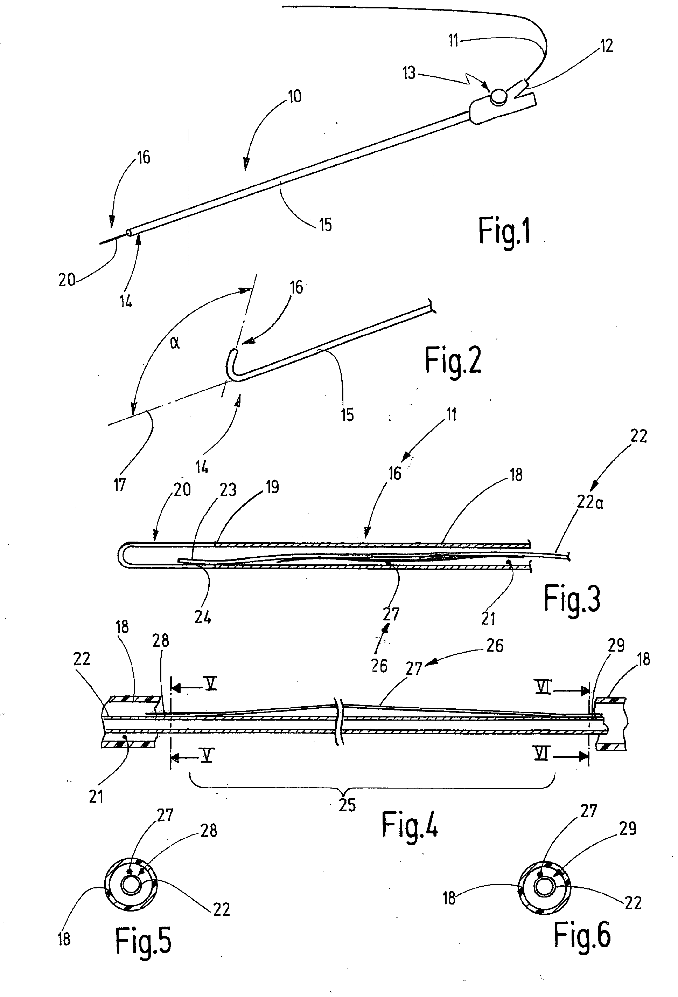

In Figur 1 ist ein Endoskop 10 veranschaulicht, in das eine Kryosonde 11 eingeführt ist. Diese erstreckt sich von dem proximalen Ende 12 des Endoskops bis zu seinem über Bedienelemente 13 beweglichen distalen Ende 14. Das Endoskop 10 weist einen länglichen Schaft 15 auf, durch dessen Kanal die Kryosonde 11 geführt ist. Das distale Ende 16 der Kryosonde 11 kann in den Schaft 15 eingezogen oder aus diesem herausgeschoben werden. Der Außendurchmesser der Kryosonde 11 ist vorzugsweise etwas kleiner als der Innendurchmesser des in dem Schaft 15 vorgesehenen Kanals.

Die Bedienelemente 13 dienen dazu, das distale Ende 14 des Schafts 15 zu steuern, insbesondere gezielt gegen die Axialrichtung 17 abzuwinkeln, die, wie es Figur 2 zeigt, längs zum Schaft 15 liegt. Der dabei erzielbare Winkel α ist vorzugsweise größer als 90°, weiter vorzugsweise größer als 140° und im bevorzugten Fall größer als 160°. Der Biegeradius ist dabei bei einem Außendurchmesser des Schafts 15 von kleiner 3,3 mm kleiner als 20 mm, vorzugsweise kleiner als 15 mm.

Die Kryosonde 11 ist im Bereich ihres distalen Endes 16 in Figur 3 gesondert veranschaulicht. Sie weist einen Schlauch 18 auf, an dessen distales Ende 19 ein Kopf 20 fluiddicht angeschlossen ist. Der Schlauch 18 umschließt ein Lumen 21 und ist zumindest an dem sich an den Kopf 20 anschließenden Abschnitt hochflexibel ausgebildet. Der Kopf 20 ist in Figur 3 schematisiert veranschaulicht. Er weist vorzugsweise einen Außendurchmesser auf, der mit dem Außendurchmesser des Schlauchs 18 übereinstimmt. An seinem distalen Ende ist der Kopf 20 durch einen ebenen, gerundeten oder anderweitig ausgebildeten Boden geschlossen.

Der Schlauch ist außerdem mit einem Fluidkanal 22 versehen, der dazu dient, Kühlfluid an den Kopf 20 heran oder in diesen zu führen. Der Fluidkanal 22 kann durch ein Kapillarrohr 22a gebildet sein, das sich durch das Lumen 21 des Schlauchs 18 erstreckt. Das Kapillarrohr 22a kann an seinem kopfseitigen Ende 23 mit dem Kopf 20 verbunden sein. Die Verbindung kann durch nicht weiter veranschaulichte Verbindungselemente oder, wie aus Figur 3 ersichtlich, unmittelbar durch eine Schweißnaht 24, einen Schweißpunkt oder dergleichen erfolgen.

Das Kapillarrohr 22a kann an seinem distalen Ende offen oder mit einer Düse versehen sein. die Düse kann auch an dem Kapillarrohr angeformt sein. Vorzugsweise besteht das Kapillarrohr 22a aus einem zugfesten Stahl, beispielsweise X2CrNiMo 1.4404 oder X2CrNiMo 1.4401. Vorzugsweise weist das Material des Kapillarrohrs 22a eine Zugfestigkeit von mehr als 900 N/mm² auf. Das Kapillarrohr 22a dient der Einleitung von Kryofluid in den Innenraum des Kopfs 20 zur Kühlung desselben sowie auch zur Übertragung von Zugkräften bei der Biopsieentnahme.

Das Kapillarrohr 22a ist jedoch nicht durchgängig zugfest ausgebildet. In seinem in Figur 4 gesondert veranschaulichten Abschnitt 25 ist das Kapillarohr 22a durch eine Wärmebehandlung, beispielsweise durch Weichglühen, in seiner Zugfestigkeit und somit auch in seiner Biegesteifigkeit reduziert. Vorzugsweise ist die Zugfestigkeit in diesem Abschnitt geringer als 700 N/mm². Damit ist das Kapillarrohr 22a auch in dem Abschnitt 25 der durch das Kryofluid ausgeübten Druckbelastung gewachsen. Es ist außerdem flexibel, sodass die Kryosonde mit geringem Biegeradius abgewinkelt werden kann, wie es in Figur 2 angedeutet ist. Jedoch ist der Abschnitt 25 nicht zugfest genug, um die nötigen Zugkräfte zur Biopsieentnahme zu übertragen.

Der Abschnitt 25 ist durch ein Zugelement 26 überbrückt, das im vorliegenden Ausführungsbeispiel ein Zugdraht 27 ist. Dieser besteht aus einem zugfesten Material, das die zur Biopsieentnahme erforderlichen Zugkräfte im Zusammenspiel mit dem Kapillarrohr 22a innerhalb seiner elastischen Materialbeanspruchung überträgt. Der Zugdraht 27 ist an seinen beiden Enden durch Schweißverbindungen 28, 29, beispielsweise Schweißnähte, mit dem Kapillarrohr 22a verbunden, das die Zugkräfte ebenfalls innerhalb seiner elastischen Materialbeanspruchung überträgt. Das Zugelement 26 hält Zugbeanspruchungen jedoch weitgehend von der weichgeglühten Stelle des Kapillarrohrs 22a fern. Der entlang der Länge des Kapillarrohrs 22a gemessene Abstand der Schweißnähte 28, 29 voneinander ist jedenfalls größer als die Länge des in seiner Zugfestigkeit und Biegesteifigkeit gegenüber dem sonstigen Kapillarrohr 22a reduzierten Abschnitt.

Die Figuren 5 und 6 veranschaulichen dies und lassen auch erkennen, dass die Schweißverbindungen 28, 29 an den beiden Enden des Zugdrahts 27 an der gleichen Radialposition des Kapillarrohr 22a angeordnet sind. Zwischen den beiden Schweißverbindungen 28, 29 liegt der Zugdraht 27 ungespannt (schlaff) und seitlich beweglich an dem Kapillarrohr 22a an oder verläuft in geringem Abstand zu diesem, wie es Figur 3 andeutet. In der Ausrichtung ist der Zugdraht 27 weitgehend parallel zum Kapillarrohr 22a orientiert.

Zur Biopsieentnahme wird die Kryosonde 11 mit dem Endoskop 10 in ein Lumen des Patienten eingeführt und das distale Ende 16 mit dem zu beprobenden Gewebe des Patienten in Berührung gebracht oder in dieses eingestochen. Das Einführen der Kryosonde 11 in das Endoskop 10 wird insbesondere dadurch erleichtert, dass die Kryosonde 11 entsprechend der Steifigkeit des Kapillarrohrs 22a fast entlang ihrer gesamten Länge steif, d.h. steifer als der Abschnitt 25, ist. Das Instrument ist jedoch insgesamt flexibel. Lediglich der von dem Abschnitt 25 bestimmte Teil der Länge ist biegeweicher und leichter abwinkelbar.

Das Endoskop kann, wie Figur 2 zeigt, falls erforderlich, um einen Winkel von über 160° abgewinkelt werden. Dadurch können das Endoskop 10 und die Kryosonde 11 in enge und stark verwinkelte Gefäße eines Patienten eingeführt werden. Die Kryosonde 11 ist dabei so bemessen, dass der Abschnitt 25 im Bereich der Biegestelle des Endoskops liegt. Die Länge des Abschnitts 25 ist dabei vorzugsweise so groß, dass das Abwinkeln sowohl dann möglich ist, wenn der Kopf 20 noch an der Öffnung des distalen Endes 14 des Schafts 15 steht, wie auch, wenn der Kopf 20, wie Figur 1 und 2 zeigen, aus dem Schaft 15 herausgeschoben ist. Vorzugsweise beträgt die Länge des Abschnitts 25 mehr als einige Zentimeter, vorzugsweise mehr als 10 cm. Die Biegung des Endoskops 10 wird durch die Steifigkeit des Kapillarrohrs 22a nur geringfügig behindert, weil der Abschnitt 25 entsprechend biegeweich ausgeführt ist. Der Schlauch 18 ist ebenfalls aus einem biegeweichen Material ausgeführt, vorzugsweise einem Kunststoff, vorzugsweise einem PEEK oder PA, welches die Biegung des Endoskops nur geringfügig behindert. Der zugsteife Zugdraht 27 setzt der Biegung aufgrund seines geringen Durchmessers ebenfalls keinen nennenswerten Widerstand entgegen. Der Durchmesser des Zugelements 26, insbesondere des Zugdrahts 27, ist geringer als der Durchmesser des Kapillarrohrs 22a.

Zur Probenentnahme wird der Kopf 20 innen mit Kryofluid beaufschlagt, welches durch das Kapillarrohr 22a in den Kopf 20 eingeführt wird. Durch die Abkühlung des Kopfs 20 frieren Teile des zu beprobenden Gewebes an diesem an.

Zur Probenentnahme wird die Kryosonde 11 in proximaler Richtung bewegt. Dabei wird das an den Kopf 20 angefrorene Gewebe von dem übrigen Gewebe abgerissen. Die dazu nötige Kraft wird über das Kapillarrohr 22a zunächst bis an die Schweißverbindung 29 und von dieser über den Zugdraht 27 auf die Schweißverbindung 28 übertragen. Von dort geht der Kraftfluss über das Kapillarrohr 22a zu dem Kopf 20. Kräftemäßig überbrückt der Zugdraht 27 somit den nicht zugfesten Abschnitt 25.

Weiter ist es möglich, das Zugelement 26 lediglich an der Verbindungsstelle 28 mit dem Kapillarrohr 22a zu verbinden und es durch die gesamte Länge der Kryosonde 11 bis an deren proximales Ende zu führen. In diesem Fall kann das Kapillarrohr 22a vom proximalen Ende bis zu der Verbindungsstelle 28 vollständig oder in einem oder in mehreren Abschnitten aus einem metallischen oder nicht metallischen Material ausgebildet sein, das flexibler ist, als das übrige Kapillarrohr 22a.

Weiter ist es möglich, das distale Ende des Zugelements 26 direkt mit dem Kopf 20 zu verbinden, während das proximale Ende des Zugelements über die Schweißverbindung 29 oder eine sonstige Verbindung mit dem Kapillarrohr 22a verbunden ist. In diesem Fall kann das Kapillarrohr 22a ausgehend von der Schweißverbindung 29 oder einer sonstigen Verbindungsstelle bis zu seinem distalen Ende ganz oder teilweise aus einem Material ausgebildet sein, das flexibler und weniger zugfest ist, als das übrige Kapillarrohr 22a.

Es ist außerdem möglich, das Kapillarrohr 22a insgesamt oder in einem oder in mehreren Abschnitten aus einem flexiblen nicht biegesteifen und nicht zugfesten Material auszubilden. In diesem Fall ist das Zugelement 26 an seinem distalen Ende an den Kopf 20 oder ein mit diesem verbundenes Element angeschlossen, während sein proximales Ende mit dem proximalen Ende der Kryosonde 11 verbunden ist.

Es wird darauf hingewiesen, dass als Zugelement 26 anstelle eines Zugdrahts 27 auch ein zugfestes Metallband, ein Drahtbündel, ein Seil, ein Rohr oder dergleichen Anwendung finden kann. Auch kann anstelle eines metallischen Zugelements 26 ein nicht metallisches Zugelement Anwendung finden, dessen Enden ebenfalls mit dem Kapillarrohr 22a verbunden sind, um wenigstens den Abschnitt 25 oder längere Teile des Kapillarrohrs 22a oder das gesamte Kapillarrohr 22a zu überbrücken. Das Zugelement 26 kann auch als Monofil oder als Seil aus einem nicht metallischen Material oder aus einem Verbundwerkstoff, zum Beispiel einem Faserverbundwerkstoff, ausgebildet sein.

Bei einer bevorzugten Ausführungsform ist das Kapillarrohr lediglich in seinem Abschnitt 25 biegeweich. Dieser Abschnitt 25 hat typischerweise eine Länge von 10 cm bis 30 cm und ist auf eine Länge begrenzt, die sich als Summe der Länge des Aktivabwinkelbereichs des Endoskops und der maximalen Ausfahrlänge der Kryosonde 11 aus dem Endoskop 10 während der Anwendung ergibt. Die Länge des Zugelements 26 ist dabei so bemessen, dass zumindest die gesamte Länge des biegeweichen Bereichs 25 des Kapillarrohrs 22a überbrückt wird. Das Zugelement 26 ist dabei proximal kraftübertragend an dem Kapillarrohr 22a und distal an dem Kopf 20 oder an dem Kapillarrohr 22a verankert, sofern sich der biegeweiche Abschnitt nicht bis zu dieser Stelle erstreckt. Weil das Kapillarrohr 22a lediglich in dem Abschnitt 25 weich, ansonsten aber steif ist, lässt sich die Kryosonde 11 in gewohnter Weise leicht handhaben. Außerdem wird sichergestellt, dass das Zugelement 26 den Querschnitt des Lumens 21 nur auf kurzer Länge des Schlauchs 18 einschränkt und somit den Strömungswiderstand in diesem nicht wesentlich reduziert.

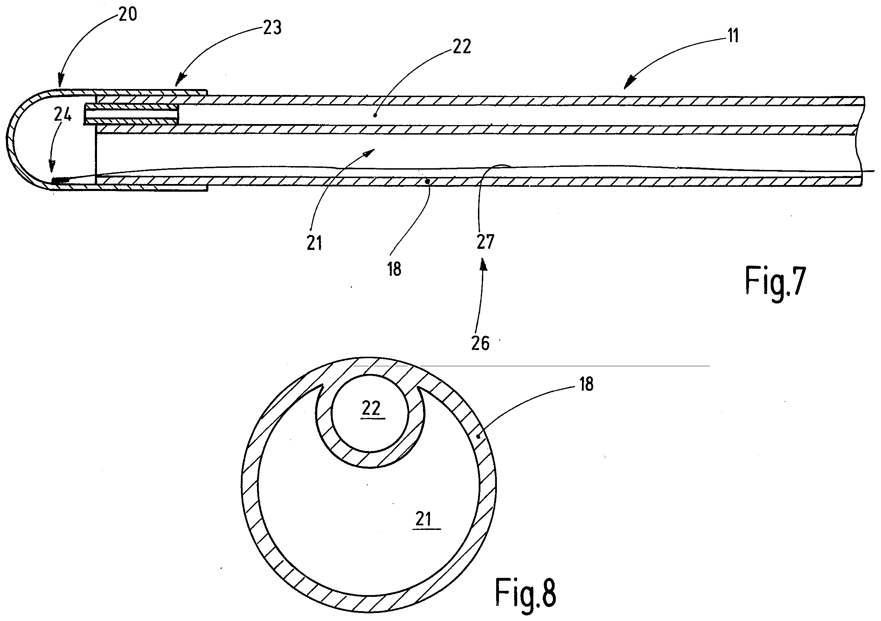

Figur 7 veranschaulicht eine abgewandelte Ausführungsform des Instruments 11, für das unter Zugrundelegung der eingeführten Bezugszeichen die vorige Beschreibung entsprechend gilt. Jedoch ist das Instrument 11 nach Figur 7 gegenüber dem Instrument 11 nach den Figuren 3 bis 6 abgewandelt. Sein Schlauch 18 ist zweilumig ausgebildet, indem parallel zu dem Lumen 21 der Fluidkanal 22 angeordnet ist. Figur 8 veranschaulicht dazu im vergrößerten Querschnitt eine beispielhafte Kanalanordnung. Während der Fluidkanal 22 zum Beispiel einen kreisförmigen Querschnitt aufweisen kann, kann der Querschnitt des Lumens 21 von der Kreisform abweichend ausgebildet sein, wie es Figur 8 veranschaulicht oder ebenfalls kreisförmig sein.

Bei der Ausführungsform des Instruments 11 nach den Figuren 7 und 8 ist dem Schlauch 18 wiederum ein Zugelement 26 zugeordnet, beispielsweise in Form eines Zugdrahtes 27, der sich zum Beispiel durch das Lumen 21 erstrecken und mit seinem distalen Ende an einer Schweißnaht 24 mit dem Kopf 20 verbunden sein kann. Der Zugdraht 27 kann sich bis zu dem proximalen Ende des Schlauchs 18 erstrecken, um von dort aus Zugkräfte auf den Kopf 20 zu übertragen.

Als Zugelement 26 kommen weitere zugfeste Elemente in Betracht, wie beispielsweise Bänder, Profildrähte, Drahtbündel, Seile oder dergleichen. Das Material des Zugelements 26 kann Metall oder auch ein Nichtmetall wie beispielsweise Kohlenstofffasern, Aramidfasern oder dergleichen sein. Das Zugelement 26 kann alternativ in die Wandung des Schlauchs 18 eingebettet sein. Das Zugelement 26 ist dabei jedenfalls vorzugsweise bei allen Ausführungsformen nach Figur 3 bis Figur 8 in Längsrichtung des Instruments 11 angeordnet, wobei das Zugelement 26 vorzugsweise gerade angeordnet ist. Das Zugelement umschlingt vorzugsweise weder das Lumen 21 noch den Fluidkanal 22, sondern ist im Wesentlichen parallel zu diesen angeordnet.

Eine erfindungsgemäße Kryosonde weist einen Kopf 20 auf, dem über ein Kapillarrohr 22a Kryofluid zugeführt wird. Zur Abfuhr des Kryofluids dient ein Schlauch 18, durch dessen Lumen 21 sich das Kapillarrohr 22a erstreckt. Das Kapillarrohr 22a weist einen biegeweichen Abschnitt 25 auf, der von einem Zugelement 26 überbrückt wird. Auf diese Weise wird eine gut handhabbare Kryosonde 11 erhalten, die sich leicht und sehr weit abwinkeln lässt und dennoch die zur Probeentnahme nötigen Zugkräfte überträgt.

### Bezugszeichen:

- 10: Endoskop
- 11: Kryosonde
- 12: proximales Ende des Endoskops 10
- 13: Bedienelemente
- 14: distales Ende des Endoskops 10
- 15: Schaft
- 16: distales Ende der Kryosonde 11
- 17: Längsrichtung des Schafts 15
- α: Winkel
- 18: Schlauch
- 19: distales Ende des Schlauchs 18
- 20: Kopf
- 21: Lumen des Schlauchs 18
- 22: Fluidkanal
- 22a: Kapillarrohr
- 23: kopfseitiges Ende des Kapillarrohrs 22a
- 24: Schweißnaht
- 25: Abschnitt
- 26: Zugelement
- 27: Zugdraht
- 28, 29: Schweißverbindungen

## Patentansprüche

1. Kryosonde (11), insbesondere zur Gewebeprobenentnahme im oberen Harntrakt eines Patienten,
mit einem Schlauch (18), der ein Lumen (21) aufweist und an seinem distalen Ende (19) mit einem Kopf (20) versehen ist,
mit einem sich durch das Lumen (21) des Schlauchs erstreckenden Kapillarrohr (22a) aufweisend einen Fluidkanal (22) der an seinem distalen Ende (23) mit dem Kopf (20) kommunizierend angeordnet ist, wobei das Kapillarrohr (22a) aus einem flexiblen, nicht biegesteigen und nicht zugfesten Material ausgebildet ist,
mit mindestens einem Zugelement (26), das in dem Schlauch (18) sich über wenigstens einen Teil seiner Länge erstreckend angeordnet ist und das an seinem distalen Ende an den Kopf (20) oder ein mit dem Kopf (20) verbundenes Element angeschlossen ist.

2. Kryosonde nach einem der vorstehenden Ansprüche, wobei ein proximales Ende des Zugelements (26) mit dem proximalen Ende der Kryosonde (11) verbunden ist.

3. Kryosonde nach einem der vorstehenden Ansprüche, wobei das Zugelement (26) zur Übertragung von Zugkräften von dem Kopf (20) auf ein proximales Ende der Kryosonde (11) dient.

4. Kryosonde nach einem der vorstehenden Ansprüche, wobei das Kapillarrohr (22a) an seinem distalen Ende mit einer Düse versehen ist.

5. Kryosonde nach einem der vorstehenden Ansprüche, wobei der Kopf (20) einen Außendurchmesser aufweist, der mit dem Außendurchmesser des Schlauchs (18) übereinstimmt.

6. Kryosonde nach einem der vorstehenden Ansprüche, wobei der Kopf (20) an seinem distalen Ende durch einen gerundeten Boden geschlossen.

7. Kryosonde nach einem der vorstehenden Ansprüche, wobei das Zugelement (26) durch ein oder mehrere Drähte aus hochfestem Stahl, insbesondere Edelstahl, mit einer Zugfestigkeit >900N/mm² gebildet ist.

8. Kryosonde nach einem der vorstehenden Ansprüche, wobei das Zugelement (26) weitgehend parallel zu dem Kapillarrohr (22a) angeordnet ist.

9. Kryosonde nach einem der vorstehenden Ansprüche, wobei in dem Lumen (21) nur ein Zugelement (26) angeordnet ist.

10. Kryosonde nach einem der vorstehenden Ansprüche, **dadurch gekennzeichnet, dass** der Durchmesser des Zugelements (26) geringer ist, als die Differenz zwischen dem Durchmesser des Lumens (21) und dem Außendurchmesser des Kapillarrohrs (22a).

11. Kryosonde nach Anspruch 1, **dadurch gekennzeichnet, dass** der Schlauch (18) zweilumig ausgebildet ist, dessen beide Lumen das Lumen (21) und der Fluidkanal (22) sind, wobei sich das Zugelement (26) durch das Lumen (21) oder den Fluidkanal (22) oder durch die Wandung des Schlauchs (18) erstreckt.
